(19) Europäisches Patentamt European Patent Office Office européen des brevets

(11) **EP 3 164 013 B1**

(12) **FASCICULE DE BREVET EUROPEEN**

(45) Date de publication et mention
de la délivrance du brevet:
**21.11.2018 Bulletin 2018/47**

(51) Int Cl.:
***A23G 4/10*** *(2006.01)*

(21) Numéro de dépôt: **15753088.2**

(22) Date de dépôt: **01.07.2015**

(86) Numéro de dépôt international:
**PCT/FR2015/051817**

(87) Numéro de publication internationale:
**WO 2016/001586 (07.01.2016 Gazette 2016/01)**

(54) **NOUVELLE COMPOSITION DE PRODUIT DE CONFISERIE**

NEUARTIGE ZUSAMMENSETZUNG VON SÜSSWAREN

NOVEL COMPOSITION OF A CONFECTIONERY PRODUCT

(84) Etats contractants désignés:
**AL AT BE BG CH CY CZ DE DK EE ES FI FR GB
GR HR HU IE IS IT LI LT LU LV MC MK MT NL NO
PL PT RO RS SE SI SK SM TR**

(30) Priorité: **01.07.2014 FR 1456288**

(43) Date de publication de la demande:
**10.05.2017 Bulletin 2017/19**

(73) Titulaire: **Roquette Frères
62136 Lestrem (FR)**

(72) Inventeurs:
• **BUSOLIN, André
F-59130 LAMBERSART (FR)**
• **BARRE, Antoine
F-59350 Saint-André-lez-Lille (FR)**

(74) Mandataire: **Cabinet Plasseraud
66, rue de la Chaussée d'Antin
75440 Paris Cedex 09 (FR)**

(56) Documents cités:
**EP-A2- 0 347 121        US-A1- 2013 177 668
US-A1- 2013 302 387**

• **Anonymous: "Sucre glace - Wikipédia", , 28 mai
2014 (2014-05-28), XP055217333, Extrait de
l'Internet:
URL:https://fr.wikipedia.org/w/index.php?t
itle=Sucre_glace&oldid=104173601 [extrait le
2015-09-30]**
• **Rowe Et Al.: "Handbook of Pharmaceutical
excipients 7th Edition" In: "Handbook of
Pharmaceutical excipients 7th Edition", 1 janvier
2012 (2012-01-01), Pharmaceutical Press,
XP055217505, pages 479-482, page 479 - page 480**

Remarques:
Le dossier contient des informations techniques
présentées postérieurement au dépôt de la demande
et ne figurant pas dans le présent fascicule.

**Description**

DOMAINE DE L'INVENTION

La présente invention concerne une nouvelle composition de produit de confiserie ou pharmaceutique caractérisée en ce qu'elle comprend entre 30% et 70% en poids d'un agent de charge différent du maltitol possédant une surface spécifique inférieure à 0,5 m²/g et possédant également une quantité de gomme de base réduite, sans effet négatif sur les propriétés organoleptiques, par rapport aux produits de l'art antérieur. L'invention concerne également le procédé de mise en oeuvre de ladite composition dans la fabrication d'un chewing-gum caractérisé en ce qu'il permet de réduire considérablement la quantité de gomme de base desdits produits.

ARRIERE-PLAN TECHNOLOGIQUE

Les hommes connaissent depuis bien longtemps le mâchage, bien avant l'apparition du chewing-gum. En effet, les hommes préhistoriques mâchaient déjà de la sève de conifère, des feuilles, des sécrétions de plantes, des racines. Au Mexique, les Mayas, il y a plus de 3000 ans, mâchaient de la sève de sapotier, sorte de latex appelé « chiclé ». En 400 avant J.C, les Grecs mâchaient de la résine, les Indiens d'Amazonie des boulettes de tabac, ou de la chique de coca extrait des petits arbustes péruviens : le kolatier.

Pourtant, il fallut attendre le XIXème siècle pour qu'apparaisse le chewing-gum tel que nous le connaissons aujourd'hui.

En 1869, le brevet du chewing-gum est déposé par le dentiste William Finley Semple, convaincu de ses effets bénéfiques pour les dents, mais il ne commercialisera pas son invention. C'est le new-yorkais Thomas Adams qui en aura l'idée vers 1870 en mettant au point une machine à produire du chewing-gum. C'est lui, qui, en mélangeant du chiclé, de la résine et du sirop, fabrique et commercialise en 1872 les premiers chewing-gums.

Aujourd'hui, la France est devenue le 2ème pays consommateur mondial de chewing-gum derrière les Etats-Unis. Le chewing-gum peut être consommé à tout moment de la journée. Il est le produit idéal lorsqu'on a envie de consommer quelque chose d'agréable ou de manger une sucrerie. De plus, le chewing-gum, quel que soit son parfum, procure une haleine fraîche et joue un rôle hygiénique et social. 53% des personnes mâchent du chewing-gum pour rafraîchir l'haleine. Le chewing-gum apparaît de plus en plus comme un substitut au dentifrice. 39% des personnes mâchent du chewing-gum pour nettoyer leurs dents quand ils ne peuvent pas les brosser. Le chewing-gum est notamment consommé après le repas car il facilite la digestion en favorisant la sécrétion salivaire et le travail de l'estomac. Beaucoup de consommateurs utilisent le chewing-gum comme un anti-stress ou comme un moyen de réduire leur tension nerveuse et de se détendre. 30% des personnes aiment mâcher du chewing-gum quand ils sont énervés et 27% se calment en mâchant du chewing-gum. Le chewing-gum est également considéré comme un substitut efficace à la cigarette. A une époque où les mesures légales visant à réduire la consommation de tabac se développent fortement, le chewing-gum a encore de belles perspectives de développement.

Le chewing-gum (ou gomme à mâcher, pâte à mâcher, chiclette) est une gomme à laquelle sont ajoutés des arômes et parfums alimentaires, destinée à être mâchée. Tous les chewing-gums sont fabriqués à partir d'une gomme de base à laquelle on rajoute des arômes et du sucre et/ou des édulcorants pour donner le goût. Le chewing-gum est un mélange de deux phases : une phase liquide (sirop, sucres et/ou édulcorant dilués) et une phase solide composée de la gomme de base et de sucre et/ou édulcorant cristallisé.

Actuellement, le chiclé, gomme de base naturelle issue du tronc des Sapotiers mais devenue trop chère du fait de la rareté des arbres et des coûts de production et de transport trop élevés), a été substituée par un produit de synthèse (la gomme de base) qui se compose de :

- 1 ou 2 élastomères qui déterminent l'élasticité,

- des cires abaissant le point de ramollissement et possédant un pouvoir anti-collant et plastifiant,

- des charges minérales qui améliorent les qualités mécaniques,

- un antioxydant qui protège les qualités de la gomme lors de la fabrication et qui la protège du vieillissement,

- des résines qui assurent le liant des matières premières de la gomme.

Le dosage de ces 5 ingrédients détermine le type de gomme (chewing-gum ou bubble-gum). La recette reste souvent secrète car celle-ci n'est pas constante. Elle varie en fonction du prix de la matière première. Les ingrédients constitutifs de la gomme de base sont insolubles dans l'eau. Par contre, la majorité des ingrédients constitutifs des chewing-gums, à l'exception de la gomme de base, est soluble dans l'eau (c'est-à-dire la salive ici). Au bout de 3 à 4 minutes de temps de mastication, les composés sont extraits (solubilisés) par la salive, d'où la perte de goût du chewing-gum. Il reste en bouche la gomme de base et quelques arômes qui ne sont pas solubles dans l'eau.

La gomme de base est un produit complexe à fabriquer : les ingrédients sont dosés avec rigueur pour obtenir des gommes plus ou moins élastiques. Les ingrédients sont malaxés entre une heure et demie et deux heures dans un pétrin qui fonctionne comme celui des boulangers. Le malaxage fait chauffer la gomme. Elle atteint au final une température de 95°C à 98 C. L'élastomère utilisé (à la place du chiclé) est un copolymère isobutylène-isoprène (butyl) de qualité alimentaire.

On ajoute à cette base les arômes, les édulcorants ou le sucre ainsi que divers additifs et auxiliaires de fabrication (colorant, gélatine, émulsifiant, stabilisant, agent gélifiant, bicarbonate, cire de carnauba). Les ingrédients et la gomme de base sont mélangés dans un pétrin pendant 15 à 20 minutes. En fin de malaxage, la pâte atteint une température de 50 °C environ. La pâte de chewing-gum est déposée à l'intérieur d'une extrudeuse. Bien pressée, elle forme maintenant des bandes plus ou moins épaisses. Les bandes passent ensuite dans le laminoir et sont découpées en tablettes ou en noyaux appelés également centres. Après refroidissement, les tablettes ou les centres sont maintenus à une température et une humidité contrôlées pendant 6 à 48 heures. Cette phase est très contrôlée, car la qualité des gommes à mâcher en dépend.

Les tablettes sont enveloppées dans un emballage en aluminium pour conserver toute leur saveur. Elles sont ensuite mises en paquets. Les centres sont dragéifiés avant d'être emballés dans des contenants en carton ou en plastique.

Quel que soit l'âge des consommateurs, la volonté d'avoir des produits de qualité est permanente. La qualité des chewing-gums se mesure par plusieurs paramètres, dont la texture du chewing-gum (plutôt dur ou au contraire plutôt mou, croustillance persistante des dragées lors de la mastication) et le goût (saveur sucrée, effet rafraîchissant ou non, persistance de l'arôme lors de la mastication). En effet, les consommateurs se plaignent très souvent de la disparition trop rapide à la fois de la croustillance et du goût lors de la mastication.

De plus, dans une volonté permanente de réduction des coûts, les industriels cherchent constamment des améliorations de leurs recettes déjà existantes sans pour autant impacter les qualités organoleptiques des produits finis. Ces réductions de coûts recherchées passent par exemples par la réduction des ingrédients chers, comme la gomme de base et/ou la quantité d'arôme utilisés.

De nombreux travaux de recherche ont déjà été menés sur la persistance du goût par de nombreuses sociétés. La Demanderesse a également travaillé sur ce sujet et on peut à ce titre citer le brevet EP 0664960B dans lequel la Demanderesse a démontré qu'il était possible d'améliorer la qualité organoleptique d'un chewing-gum, et notamment améliorer le goût et l'aromatisation en termes d'impact et de durée, en lui faisant comporter, en tant que phase pulvérulente, du maltitol ayant une pureté en maltitol supérieure à 95% et une granulométrie telle que 50% des particules de maltitol au sein du chewing-gum soient de taille inférieure à 90 microns. Le brevet EP0347121 décrit une composition de chewing-gum comprenant un agent de charge consitué de mannitol et de sorbitol co-crystallisés.

Désireuse d'améliorer encore l'état de la technique et surtout de répondre aux attentes toujours plus exigeantes des consommateurs, la Demanderesse s'est donc attachée à l'obtention d'un nouveau chewing-gum présentant toutes les caractéristiques recherchées avec une quantité de gomme de base diminuée dans le produit fini sans aucun impact sur les qualités organoleptiques, et notamment sur le volume de mâche et/ou sur la note aromatique perçue lors de la mastication par les consommateurs.

RESUME DE L'INVENTION

Après de nombreux travaux de recherche, la Demanderesse a constaté que l'on pouvait, de façon surprenante et inattendue, obtenir un chewing-gum possédant toutes les caractéristiques organoleptiques d'un chewing-gum de l'art antérieur, en utilisant un agent de charge différent du maltitol possédant une surface spécifique particulière et, de préférence, une porosité donnée.

L'utilisation de cet agent de charge bien particulier permet, entre autre, de réduire la quantité de gomme de base utilisée dans la mise en oeuvre des compositions de chewing-gums.

Ainsi l'invention concerne une composition de chewing-gum caractérisée en ce qu'elle comporte entre 30% et 70% en poids d'un agent de charge différent du maltitol possédant une surface spécifique déterminée par la méthode BET inférieure à 0,5 $m^2$/g, de préférence comprise entre 0,1 et 0,45 $m^2$/g, de préférence comprise entre 0,2 et 0,45 $m^2$/g, et de préférence entre 0.2 et 0.3 $m^2$/g, ladite surface spécifique étant mesurée sur une coupe de 250$\mu$m à 841 $\mu$m, le dit agent de charge étant le sorbitol.

Selon l'invention, cette composition de chewing-gum est également caractérisée en ce que l'agent de charge possède, de préférence, une porosité inférieure à 0,0085 ml/g, de préférence inférieure à 0,0080 ml/g et encore plus préférentiellement inférieure à 0,0070 ml/g. Ledit agent de charge est compris entre 30% et 70%, de préférence entre 40% et 60%, et plus préférentiellement encore entre 45% et 55% dans la composition de chewing-gum selon l'invention, les pourcentages étant exprimés en poids par rapport au poids total de la composition de chewing-gum mise en oeuvre.

Toujours selon la présente invention, ladite composition est également caractérisée en ce que l'agent de charge différent du maltitol est une composition pulvérulente de sorbitol.

Ledit agent de charge est le sorbitol, plus préférentiellement présentant une pureté supérieure à 95% en poids sec de sorbitol.

La composition de chewing-gum selon l'invention comprend :

- de 10% à 28%, préférentiellement de 15% à 25%, et encore plus préférentiellement 20% d'au moins une gomme de base,
- de 30% à 70%, de préférence de 40% à 60%, et plus préférentiellement encore de 45% à 55% d'un agent de charge différent du maltitol possédant une surface spécifique faible, c'est-à-dire inférieure à 0,5 $m^2$/g, mesurée sur une coupe de 250$\mu$m à 841$\mu$m,
- de 0,1 % à 5 %, préférentiellement de 0,5 % à 3 %, et encore plus préférentiellement de 1 à 1,8 % d'au moins un arôme,

les pourcentages étant indiqués en poids sec par rapport au poids sec total de ladite composition, le dit agent de charge étant le sorbitol.

## DESCRIPTION DETAILLEE DES MODES DE REALISATION

La présence invention concerne une nouvelle composition de chewing-gum caractérisée en ce que la dite composition de chewing-gum comporte entre 30% et 70% en poids d'un agent de charge différent du maltitol possédant une surface spécifique déterminée par la méthode BET inférieure à 0,5 $m^2$/g, de préférence comprise entre 0,2 et 0,45 $m^2$/g, ladite surface spécifique étant mesurée sur une coupe de 250$\mu$m à 841 $\mu$m, le dit agent de charge étant le sorbitol.

Dans toute la présente invention, on considérera que tous les pourcentages exprimés, sauf mention contraire explicite, le sont par rapport au poids total de la composition de chewing-gum mise en oeuvre.

La surface spécifique d'une poudre correspond à la surface développée par unité de masse, porosité ouverte comprise. Elle rend compte de la forme des particules et de la rugosité de leur surface. La méthode de mesure de la surface spécifique est bien connue par l'homme du métier et largement documentée dans les ouvrages de référence. L'aire massique d'un solide, ici l'agent de charge, est déterminée après dégazage, par adsorption d'une monocouche de gaz, par liaison de Van Der Waals, autour de chaque particule et dans chaque pore ouvert de l'échantillon. Les résultats obtenus sont exploités selon l'équation établie par Brunauer, Emmet et Teller.

On détermine la surface spécifique de la composition édulcorante selon l'invention grâce à un analyseur de surface spécifique de marque BECKMAN-COULTER, de type SA3100 basé sur un test d'absorption de l'azote sur la surface du produit soumis à l'analyse, en suivant la technique décrite dans l'article BET Surface Area by Nitrogen Absorption de S. BRUNAUER et al. (Journal of American Chemical Society, 60, 309, 1938).

L'analyse BET est réalisée en 3 points.

Par définition, la surface spécifique (*Ss*) appelée aussi « Aire massique » représente la surface totale (As) par unité de masse (M) et on l'exprime généralement en $m^2$/g.

La surface spécifique désigne la superficie réelle de la surface d'un objet par opposition à sa surface apparente.

Selon un mode préférentiel de l'invention, la composition de chewing-gum est caractérisée en ce que l'agent de charge possède une surface spécifique déterminée par la méthode BET inférieure à 0,5 $m^2$/g, de préférence comprise entre 0,2 et 0,45 $m^2$/g, et de préférence entre 0,2 et 0.3 $m^2$/g, ladite surface spécifique étant mesurée sur une coupe de 250$\mu$m à 841 $\mu$m, le dit agent de charge étant le sorbitol.

Selon ce mode préférentiel, l'agent de charge avant d'être soumis à une analyse de sa surface spécifique subit un tamisage sur des tamis dont la granulométrie est comprise entre 250 $\mu$m et 841 $\mu$m. Cela permet d'éliminer toutes les particules dont le diamètre est inférieur à 250 $\mu$m et également toutes les particules dont le diamètre est supérieur à 841 $\mu$m.

Cela permet par conséquent de se concentrer sur la coupe granulométrique concernant la distribution majoritaire des poudres d'agent de charge et de s'affranchir des fines et des particules trop grosses qui fausseraient l'analyse.

Un autre paramètre caractéristique de la composition de chewing-gum est qu'elle comprend un agent de charge possédant de préférence une porosité inférieure à 0,0085 ml/g, de préférence inférieure à 0,0080 ml/g et encore plus préférentiellement inférieure à 0,0070 ml/g. Dans la présente demande, on entend par «porosité» l'ensemble des vides (pores) constituant l'agent de charge pulvérulent, ces vides étant remplis par des fluides (liquide ou gaz). C'est une grandeur physique qui conditionne les capacités d'écoulement et de rétention d'un substrat.

La porosité est aussi une valeur numérique définie comme le rapport entre le volume des vides et le volume total d'un milieu poreux.

$$\phi = \frac{V_{pores}}{V_{total}}$$

Avec:

- $\phi$ la porosité,

- $V_{pores}$ le volume des pores, et

- $V_{total}$ : le volume total de l'agent de charge, c'est-à-dire la somme du volume de solide et du volume des pores.

Selon un mode de réalisation de l'invention, la composition de chewing-gum selon l'invention est caractérisée en ce que l'agent de charge est compris entre 30% et 70%, de préférence entre 40% et 60%, et plus préférentiellement encore entre 45% et 55%, les pourcentages étant exprimés en poids par rapport au poids total de la composition de chewing-gum mise en oeuvre, le dit agent de charge étant le sorbitol.

Selon l'invention, on entend par « agent de charge » ou « bulking agent », toute composition pulvérulente de sorbitol.

Selon un mode préférentiel, la composition de chewing-gum selon l'invention est acariogène.

Dans la présente invention, on entend par « acariogène », les compositions de chewing-gum qui n'induisent pas de caries lorsqu'elles sont consommées.

De manière plus précise, les compositions de chewing-gum selon l'invention entrainent une moindre acidification par les bactéries de la bouche que des compositions de chewing-gum contenant des sucres classiques tels que le saccharose, le glucose ou le fructose.

L'effet acariogène est en effet dû à la présence, dans la cavité buccale, d'un grand nombre et d'une grande variété de bactéries, notamment des bactéries cariogènes (les streptocoques mutants en particulier) qui colonisent la plaque dentaire (ou film dentaire) et métabolisent et fermentent les sucres des aliments en entraînant la production d'acides, notamment d'acide lactique. Ces derniers permettent un abaissement du pH périphérique de la dent en dessous du pH fatidique de 5,7, qui a pour conséquence de dissoudre l'hydroxyapatite de l'émail dentaire et d'y créer des cavités. La dent est alors fragilisée car l'acidité importante provoque une déminéralisation (dissolution) de l'émail dentaire. Puis la carie progresse à l'intérieur de la dent et atteint la pulpe en occasionnant des douleurs.

En effet, des consommations répétées ainsi qu'un long temps de séjour en bouche des aliments riches en glucides fermentescibles (contenant sucre ou saccharose, fructose, amidon...) forment un terrain propice au développement des caries.

Dans la présente invention, on entend par « hydrate de carbone acariogène », tous les glucides non fermentescibles ou glucides non acidogènes.

Selon la présente invention, la composition de chewing-gum est caractérisée en ce que l'hydrate de carbone acariogène est du sorbitol. En effet le soribtol n'est pas susceptible d'être transformé en acide par la fermentation, et donc ne participe pas à la formation de caries. Le sorbitol n'est pas métabolisé par les bactéries de la cavité buccale et n'induit pas de production d'acide. Il n'y a donc pas de baisse du pH dans la bouche en dessous de la valeur critique de 5,7 et les risques cariogènes et érosifs n'apparaissent pas.

Ainsi les compostions de chewing-gums selon la présente invention satisfont au label Sympadent.

Du point de vue des industriels de la confiserie, une volonté très nette se dégage également. Celle de fabriquer des confiseries acariogènes, c'est-à-dire qu'elles ne causent pas de carie dentaire car les produits qu'elles renferment ne produisent pas d'acides et ne sont pas métabolisés par la flore buccale bactérienne.

Les industriels cherchent à obtenir des confiseries répondant au cahier des charges très strict de l'Association Sympadent Suisse, de façon à pouvoir apposer sur leur confiserie le label connu et reconnu de tous. Ce label, un petit bonhomme en forme de dent et son parapluie, a été créé par l'Action Sympadent pour désigner les produits respectueux de la dentition et pour servir ainsi d'indicateur au service d'un comportement qui préserve la dentition. Ces produits ne doivent être ni cariogènes, ni érosifs. Différents types de sucres sont cariogènes et ceci veut dire qu'ils sont susceptibles de provoquer des caries. Le potentiel érosif dommageable par contre dépend de la teneur en acide d'un produit.

Les produits qui arborent le label Sympadent doivent tout d'abord passer un test scientifique dit « mesure du pH par télémétrie ». Ce test est effectué par des centres d'essais indépendants. Il s'agit d'une procédure normalisée dans laquelle on mesure sur des sujets expérimentaux le pH de la plaque dentaire en plaçant des électrodes recouverts de plaque dans les espaces interdentaires. La mesure se fait pendant la consommation de la confiserie à tester et trente minutes après sa consommation. La confiserie est considérée non cariogène si le pH ne tombe pas en dessous du seuil critique de 5,7. Le potentiel érosif est déterminé à l'aide d'une électrode sans plaque placée dans la salive. Les produits qui exposent les dents à moins de 40 $\mu$mol d'acide pendant leur consommation sont réputés non érosifs.

La dent qui sourit sous son parapluie est un symbole intelligible partout dans le monde. Il est compris sans autre explication. Les produits qui en sont porteurs ménagent les dents. Ce pictogramme ainsi que les indications normalisées des valeurs nutritives contribuent à une alimentation saine et respectueuse de la dentition. Un consommateur aura plutôt tendance à acheter des confiseries portant ce logo.

La Demanderesse a dans le passé concentré ses efforts sur la mise au point d'un nouveau sorbitol de surface spécifique faible, également susceptible de convenir dans les compositions de chewing-gum selon l'invention.

La Demanderesse a en effet protégé dans la demande FR 14 56288 une composition édulcorante caractérisée en ce qu'elle présente de 80 à 95% en poids sec de sorbitol pulvérulent cristallisé, une enthalpie au plus égale à 150 J/g, un diamètre moyen volumique compris entre 200 et 350 $\mu$m. La composition édulcorante est également caractérisée en ce qu'elle possède une surface spécifique, déterminée selon la méthode BET, inférieure à 0,6 m$^2$/g, de préférence comprise entre 0,15 et 0,4 m$^2$/g, et encore plus préférentiellement entre 0,20 et 0,35 m$^2$/g.

Cette composition édulcorante protégée dans la demande de brevet FR 14 56288 par la Demanderesse de pureté inférieure à 95% en poids sec de sorbitol est parfaitement apte à entrer dans les compositions de chewing-gum selon la présente invention, du fait de sa faible surface spécifique.

Ainsi, dans la présente demande, lorsque l'agent de charge est le sorbitol, il possède de préférence une pureté supérieure à 95% en poids sec de sorbitol.

L'agent de charge contenu dans la composition de chewing-gum selon l'invention est également caractérisé par sa granulométrie moyenne particulière.

On entend par granulométrie moyenne au sens de la présente invention un diamètre moyen de particules faible et inférieur à 350 micromètres. Ces valeurs sont déterminées par un granulomètre a diffraction LASER type LS 230 de la société BECKMAN-COULTER, équipé de son module de dispersion poudre (voie sèche), en suivant le manuel technique et les spécifications du constructeur. Les conditions opératoires de vitesse de vis sous trémie et d'intensité de vibration de la goulotte de dispersion sont déterminées de manière à ce que la concentration optique soit comprise entre 4 ° et 12 °, idéalement 8 °. La gamme de mesure du granulomètre à diffraction LASER type LS 230 est de 0,04 $\mu$m à 2.000 $\mu$m. Les résultats sont calculés en % volumique, et exprimés en $\mu$m. La courbe de distribution granulométrique permet également de déterminer la valeur du diamètre moyen volumique (moyenne arithmétique) D4,3.

La Demanderesse a en effet constaté que lorsque des compositions de chewing-gum contiennent comme agent de charge du sorbitol présentant une surface spécifique élevée, en particulier au moins supérieure à 1 m$^2$/g, il convenait d'employer des poudres de sorbitol possédant un diamètre moyen volumique (moyenne arithmétique) D4,3 supérieur à 100 microns mais inférieure à 350 microns. En effet, l'utilisation d'un sorbitol possédant une surface spécifique élevée et une granulométrie élevées pourront donner lors de son utilisation dans des compositions de chewing-gum une sensation de texture sableuse et donc désagréable.

Selon un mode préférentiel de la présente invention, la Demanderesse a également constaté que pour des surfaces spécifiques faibles et donc inférieures à 0,5 m$^2$/g selon la méthode BET, il était préférable que le diamètre moyen volumique (moyenne arithmétique) D4,3 de l'agent de charge de type sorbitol, soit compris entre 200 et 350 $\mu$m.

Dans un mode préférentiel, le diamètre moyen volumique (moyenne arithmétique) D4,3 de l'agent de charge de type sorbitol est compris entre 250 et 350 $\mu$m ou plus préférentiellement entre 280 et 330 $\mu$m.

Le choix de la granulométrie des poudres de sorbitol est très important. Les particules de sorbitol possèdent une structure microscopique dendritique, c'est-à-dire comme un enchevêtrement d'aiguilles. En raison de cette structure particulière, il a généralement été constaté que l'utilisation de sorbitol poudre présentant une granulométrie moyenne supérieure à 200 micromètres dans la fabrication de comprimés, tablettes et/ou chewing-gums confère auxdits produits une texture dite sableuse, notamment aux chewing-gums (en particulier lors de la mastication).

L'utilisation d'un agent de charge de type sorbitol dans la fabrication des compositions de chewing-gums selon l'invention, bien que présentant une granulométrie plus importante (une granulométrie moyenne supérieure à 200 $\mu$m) n'a pas cet effet négatif sur les qualités organoleptiques des chewing-gums. En effet, comme démontré ci-après, les chewing-gums fabriqués à partir de cet agent de charge ne présentent pas cette texture désagréable sableuse en bouche.

Le chewing-gum sans sucres est aujourd'hui numéro 1 des ventes, avec 90% de parts de marché dans la plupart des pays européens. Grâce aux polyols, les chewing-gums sont acariogènes, moins caloriques et excellents en goût.

La tendance vers une alimentation plus saine continue à gagner du terrain et modifie les modes de consommation et les habitudes d'achat de façon significative. Consommer moins de sucres tout en continuant à se faire plaisir est le souhait de plus en plus de consommateurs en réponse aux multiples recommandations nutritionnelles. L'utilisation de succédanés du sucre en remplacement du sucre se justifie pour la fabrication de denrées alimentaires à valeur énergétique réduite, de denrées non cariogènes et d'aliments sans sucre ajutés, ainsi que pour la production de produits diététiques.

Le terme polyols désigne les produits obtenus par hydrogénation catalytique de sucres réducteurs simples donc possédant un DP égal à 1 (DP = Degré de Polymérisation), mais aussi de sucres réducteurs plus complexes composés des homologues supérieurs possédant un DP supérieur ou égal à 2 de ces sucres simples, tels que les disaccharides, oligosaccharides et polysaccharides ainsi que leurs mélanges. Généralement, les sucres réducteurs simples que l'on destine à l'hydrogénation catalytique pour l'obtention des compositions de polyols du type de celles de l'invention sont le glucose, le xylose, le fructose et le mannose. Les polyols obtenus sont alors le sorbitol, le xylitol et le mannitol. Les disaccharides sont le plus souvent le maltose, le maltulose, l'isomaltulose et le lactose, qui conduisent par hydrogénation, à l'isomalt, à l'isomaltitol et au lactitol. Les oligosaccharides et polysaccharides, qui sont des produits de poids moléculaires plus élevé, proviennent d'ordinaire d'une hydrolyse acide et/ou enzymatique d'amidons et/ou de fécules, de xylanes ou de fructanes comme l'inuline, mais peuvent aussi être obtenus par recombinaison acide et/ou enzymatique de mono ou disaccharides tels que ceux cités plus haut.

Par conséquent, le terme polyol désigne un polyol choisi notamment dans le groupe comprenant le sorbitol, le xylitol, l'érythritol, l'isomalt, l'isomaltitol, le lactitol, l'alpha-D-glucopyranosyl-1-6-sorbitol (=1,6-GPS), l'alpha-D-glucopyranosyl 1-1-mannitol (=1,1-GPM), l'alpha-D-glucopyranosyl-1-1-sorbitol (=1,1-GPS) et leurs mélanges.

La Demanderesse a eu le mérite de constater que l'utilisation d'un agent de charge différent du maltitol possédant une surface spécifique faible, c'est-à-dire inférieure à 0,5 m$^2$/g, mesurée sur une coupe de 250$\mu$m à 841$\mu$m permettait de réaliser une composition de chewing-gum possédant une réduction de gomme de base non négligeable.

La Société Demanderesse a démontré que l'utilisation d'un agent de charge possédant une surface spécifique faible selon l'invention avait un intérêt particulier en termes de réduction des coûts de formulation dans une recette de chewing-gum.

Cette surface spécifique faible permet en effet de fabriquer des chewing-gum dont le volume en bouche perçu par le consommateur a été jugé similaire à un chewing-gum traditionnel, alors que le chewing-gum selon l'invention comporte 30% de moins en poids de gomme de base dans sa recette.

Ainsi selon l'invention, ladite composition de chewing-gum est caractérisée en ce que la quantité de gomme de base est réduite de 60%, de préférence de 50%, et encore de préférence de 40% par rapport à la quantité de la gomme de base comparativement à une composition de chewing-gum de l'art antérieur ou classique, sans impacter sur les qualités finales organoleptiques du produit fini, et notamment du volume perçu lors de la mâche et de l'intensité aromatique.

Qui plus est, ladite composition de chewing-gum est également caractérisée en ce que la quantité d'arômes est réduite de façon non négligeable. En effet, le fait de mettre moins de gomme de base dans la recette impacte directement sur la quantité d'arômes à ajouter.

Ainsi, ladite composition de chewing-gum est caractérisée en ce que la quantité d'arômes est réduite de 50%, de préférence de 40%, et encore de préférence de 25% par rapport à la quantité d'arômes d'une composition de chewing-gum de l'art antérieur ou classique.

Selon l'invention, l'arôme ou agent aromatisant peut comprendre des composés naturels et/ou de synthèse. Il peut s'agir en particulier d'arômes de menthe, de cannelle, d'orange, de citron, de limette ou d'arômes correspondant à d'autres fruits ou plantes tels par exemple les arômes de pomme, de fraise, de banane, de cerise ou de mélanges de fruits. L'agent aromatisant peut se présenter sous forme d'un produit unique ou sous deux ou plusieurs formes physiques différentes comprenant essentiellement les mêmes composés d'arôme. On peut employer également plusieurs agents aromatisants de natures différentes et d'états physiques identiques ou différents.

Des acides alimentaires peuvent être également ajoutés dans la composition conforme à l'invention, par exemple en tant qu'exhausteurs, à de faibles teneurs, notamment lorsqu'un arôme fruité est employé.

Dans la présente invention, le terme chewing-gum est utilisé indifféremment pour désigner les chewing-gums et les bubble-gums. La différence entre ces deux types étant d'ailleurs assez floue. On a coutume de dire que les chewing-gums se mâchent alors que les bubble gums sont destinés à faire des bulles, et donc sont traditionnellement plutôt consommés par un jeune public.

La plupart des chewing-gums, qu'ils soient avec ou sans sucre, dragéifiés ou non, comprennent essentiellement une gomme de base insoluble dans l'eau, des agents sucrants hydrosolubles apportés sous forme liquide et/ou pulvérulente et des arômes. Ils comprennent souvent d'autres ingrédients tels que des colorants, des émulsifiants, des plastifiants, des édulcorants intenses, de l'eau, etc...

La gomme de base est l'ingrédient qui différencie les chewing-gums des autres confiseries. Cette substance élastique possède la propriété de pouvoir être mâchée pendant des heures sans induire de modifications substantielles de sa texture. Elle ne se délite pas non plus au cours de la mastication. La gomme de base est un ingrédient très important dans la fabrication des centres. Elle varie en fonction du produit fini, chewing-gum ou bubble-gum, du format en sticks ou en coussinets, avec ou sans sucre etc.. Les gommes de base d'aujourd'hui sont vraiment très différentes de celles utilisées dans le passé. Elles contiennent des élastomères synthétiques, des plastifiants, des agents assouplissants ou ramollissants, texturants et émulsifiants ainsi qu'une variété d'ingrédients spécifiques qui vont lui conférer ses propriétés particulières en fonction de l'application finale.

La gomme de base constitutive de la composition de chewing-gum selon l'invention est de préférence ordinaire et semblable à celles qui sont couramment utilisées. Elle peut également comprendre des élastomères synthétiques et/ou naturels comme le polyisoprène, l'acétate de polyvinyle, le polyisobutylène, des latex, des résines comme les résines terpéniques, les esters et les alcools de polyvinyle, des matières grasses ou des cires comme par exemple la lanoline, les huiles végétales partiellement hydrogénées ou non, les acides gras, les esters partiels de glycérol, la paraffine, les cires microcristallines, des agents de charge comme du talc, le carbonate de calcium, des plastifiants d'élastomères comme le triacétate de glycérol, le monostéarate de glycérol, les dérivés de collophanes, des émulsifiants comme la lécithine, les esters de sorbitol, des colorants ou des agents de blanchiment, des antioxydants, des agents anticollants comme le mannitol.

La Société Demanderesse a notamment réussi à démontrer que l'utilisation d'un agent de charge selon l'invention, c'est-à-dire un agent de charge différent du maltitol possédant une surface spécifique faible, inférieure à 0,5 $m^2/g$, mesurée sur une coupe de 250 μm à 841 μm dans une formulation de type chewing-gum, le dit agent de charge étant le sorbitol, permet de conférer au chewing-gum une texture finale plus souple que celle des chewing-gums obtenus selon la même recette mais en mettant en oeuvre des agents de charge possédant une surface spécifique supérieure.

Etant donné que c'est la gomme de base qui permet en grande partie de conférer la texture au chewing-gum, la société Demanderesse a alors eu l'idée de réduire la quantité de gomme de base de façon à ne pas modifier la texture finale du chewing-gum. L'utilisation de l'agent de charge selon l'invention permet en outre de réduire la quantité d'arômes traditionnellement utilisée. En effet une partie des arômes reste emprisonnée dans la gomme de base lors de la mastication et ces arômes ne sont donc jamais relargués dans la salive. L'intérêt de l'utilisation d'un agent de charge possédant une surface spécifique faible, c'est-à-dire inférieure à 0,5 $m^2/g$, mesurée sur une coupe de 250 μm à 841 μm est donc double puisqu'elle permet d'une part la réduction du taux de gomme de base, ce qui permet par conséquent de diminuer d'autre part la quantité d'arômes utilisés. De telles diminutions de quantité de gomme et d'arômes engendrent une réduction importante au niveau des coûts de fabrication, et sont donc très intéressantes pour les industriels.

Les propriétés particulières de l'agent de charge utilisé confèrent la faculté d'assouplir la gomme de base et donc le chewing-gum au final.

Par ailleurs, bien que possédant une quantité moins importante d'arômes dans la recette, la perception des arômes, tant en intensité qu'en persistance, dans la composition de chewing-gum selon l'invention est au moins identique au chewing-gum selon l'art antérieur.

La société Demanderesse a notamment démontré qu'en réduisant la gomme de base, il était tout à fait possible d'obtenir des chewing-gums tout à fait satisfaisants en terme de texture. Ceci n'était pas du tout évident car les proportions entre les différents constituants sont généralement figées et qu'il n'est pas possible de les modifier sans impacter de façon négative la qualité finale des produits.

Selon un mode préférentiel, la composition de chewing-gum selon l'invention est caractérisée en ce qu'elle comprend :

- de 10% à 28%, préférentiellement de 15% à 25%, et encore plus préférentiellement 20% d'au moins une gomme de base,
- de 30% à 70%, de préférence de 40% à 60%, et plus préférentiellement encore de 45% à 55% d'un agent de charge différent du maltitol possédant une surface spécifique faible, c'est-à-dire inférieure à 0,5 $m^2/g$, mesurée sur une coupe de 250 μm à 841 μm
- de 0,1 % à 5 %, préférentiellement de 0,5 % à 3 %, et encore plus préférentiellement de 1 à 1,8 % d'au moins un arôme,

les pourcentages étant indiqués en poids sec par rapport au poids sec total de la dite composition, le dit agent de charge étant le sorbitol.

La Demanderesse recommande d'effectuer ce mélange à une température comprise entre 45°C et 80°C, préférentiellement dans un pétrin à bras en Z avec une double enveloppe ou dans un mélangeur en continu. De manière préférée, il convient de chauffer au préalable la gomme de base, à une température comprise entre 45°C et 80°C, de préférence entre 45°C et 55°C, par tout moyen connu de l'homme du métier. On pourra à titre d'exemple la chauffer dans un four à micro-ondes ou dans une étuve.

Le mélange entre les composés précités peut en outre mettre en oeuvre un autre polyol en tant qu'agent sucrant, sous forme de poudre ou de liquide, tel que par exemple le mannitol, le maltitol, le xylitol, l'erythritol, le lactitol, l'isomalt, les sirops de maltitol, les sirops de sorbitol, les sirops de glucose hydrogénés.

Le mélange entre les composés précités peut aussi mettre en oeuvre, à raison d'au plus 5 % en poids par rapport au poids total du chewing-gum, au moins un constituant choisi parmi les colorants, les édulcorants intenses tels qu'aspartame, acésulfame K, alitame, néotame, sucralose, saccharine, néohespéridine DC, stiéviosides, brazzéine..., les actifs pharmaceutiques, minéraux, extraits de plantes, anti-oxydants, et fibres indigestibles telles que par exemple les oligosaccharides tels que les fructo oligosaccharides, les fibres indigestibles telles que le Fibersol™ commercialisé par la société MATSUTANI, ou encore le NUTRIOSE® commercialisé par la Demanderesse, des émulsifiants comme la lécithine, etc...

La gomme de base utilisée pourra être adaptée au type de chewing-gum fabriqué. Elle pourra comprendre des élastomères synthétiques et/ou naturels comme le polyisoprène, l'acétate de polyvinyle, le polyisobutylène, des latex, des résines comme les résines terpéniques, les esters et les alcools de polyvinyle, des matières grasses ou des cires comme par exemple la lanoline, les huiles végétales partiellement hydrogénées ou non, les acides gras, les esters partiels de glycérol, la paraffine, les cires microcristallines.

Dans la fabrication de la composition de chewing-gum, l'étape de mélange des ingrédients précités est suivie des étapes d'extrusion, laminage, découpe, refroidissement puis conditionnement, réalisées selon toutes les techniques bien connues de l'homme du métier. Au final, le chewing-gum est présent sous une des formes bien connues de l'homme du métier, telles que tablettes, billes, dragées, cubes ou encore de comprimés.

Selon l'invention, les ingrédients et la gomme de base sont mélangés dans un pétrin pendant 15 à 20 minutes. En fin de malaxage, la pâte atteint une température de 50°C environ. Puis la pâte à mâcher est versée à l'intérieur d'une extrudeuse. Bien pressée, elle forme maintenant des bandes plus ou moins épaisses. Les bandes passent ensuite dans le laminoir et sont découpées en tablettes ou noyaux. Après refroidissement, les tablettes ou les noyaux de dragées sont maintenus à une température et une humidité précises pendant 6 à 48 heures. Cette phase est très contrôlée, la qualité des gommes à mâcher en dépend.

Selon une variante de l'invention, les compositions de chewing-gum de la présente invention peuvent être pelliculées. Le pelliculage consiste en l'application d'une composition liquide filmogène qui devient après séchage un film protecteur. Ce pelliculage sert par exemple à protéger les principes actifs contenus dans la confiserie, à protéger la confiserie elle-même de l'humidité, des chocs, de la friabilité, et également à conférer aux confiseries des propriétés visuelles attractives : brillance, couleur uniforme, surface lisse,...

Selon une variante plus préférentielle, les compositions utilisées pour le pelliculage sont celles décrites dans la demande de brevet WO2005/060944 dont la Demanderesse est titulaire.

Selon un autre mode préférentiel, les compositions de chewing-gum de la présente invention peuvent en plus, quand cela est possible, être fourrées avec des fourrages liquides, pâteux, solides, en poudre.... Elles peuvent également être enrobées de chocolat, dragéifiées, candies, givrées...

Selon un autre mode de réalisation de l'invention, la composition de chewing-gum pourra également être dragéifiée ou non. Selon l'invention, l'étape de dragéification pourra être une dragéification tendre ou une dragéification dite dure.

La dragéification dure est une opération unitaire employée dans bon nombre de domaines parmi lesquels ceux de la confiserie et de la pharmacie. Elle peut concerner également l'industrie des additifs que sont les arômes, les édulcorants, les vitamines, les enzymes, les acides et les produits à base de plantes. Cette opération consiste à créer un revêtement dur cristallin à la surface de produits solides ou pulvérulents, afin de les protéger pour diverses raisons ou bien afin de les rendre attractifs visuellement ou gustativement. Très généralement, cette opération unitaire est réalisée en plaçant de tels produits en tant que noyaux à revêtir, dans une turbine de dragéification. La dragéification dure vise à obtenir une couche croustillante et sucrée, toujours très appréciée dans le cas de confiseries ou de chewing-gums. Elle nécessite toujours l'utilisation d'un sirop et/ou d'une suspension contenant des matières cristallisables. Le revêtement dur et cristallin s'obtient alors par application de ce sirop ou de cette suspension sur les noyaux et évaporation de l'eau apportée par ceux-ci grâce à un séchage par air chaud et sec, ce qui provoque la cristallisation. Ce cycle doit être répété un très grand nombre de fois de l'ordre de dix à quatre-vingt fois afin d'obtenir le taux de grossissage voulu. On appelle couramment taux de grossissage, l'accroissement en poids des produits, considérés en fin d'opération par rapport au début, rapporté au poids final des produits.

Dans la présente invention, la dragéification dure peut être précédée ou suivie par d'autres techniques d'enrobage. On peut retenir en particulier les techniques suivantes qui se réalisent souvent aussi en employant une turbine de dragéification :

- le gommage qui est une technique dans laquelle sont utilisés des sirops de matières non cristallisables et en général non hygroscopiques comme les gommes arabiques, les amidons et les celluloses modifiés, les maltodextrines. Cette technique permet, après une ou deux applications du sirop de gommage sur le produit à revêtir, de créer un film vitreux faisant barrière à la migration de l'oxygène, de l'eau ou des matières grasses. Dans ce procédé peuvent également être employées conjointement à ces sirops non cristallisables, des poudres de diverses natures, de façon à fixer l'eau apportée par les sirops. Dans d'autres cas encore, on emploie des sucres ou des polyols fondus ou liquéfiés par des solvants. Le revêtement vitreux, dur et cassant, est obtenu alors par refroidissement ou par évaporation des solvants.

- la dragéification tendre qui consiste à créer un revêtement très souple et tendre à la surface des produits. Ce revêtement est obtenu par applications répétées d'une part d'un sirop non cristallisable comme en général les hydrolysats d'amidons, et d'autre part d'une poudre, en général du saccharose cristallisé. Le revêtement est habituellement épais. Le taux de grossissage pour cette technique est de l'ordre de 10 à 80 % voire davantage. Il est à noter que la matière constitutive du sirop est habituellement différente de celle de la poudre.

- le brillantage qui consiste par emploi de corps gras ou de cires généralement apportés sous forme cristallisée en paillettes ou de solutions alcooliques, à revêtir les produits d'une très fine pellicule grasse afin de réduire les transferts d'eau depuis ou vers les produits enrobés mais aussi d'embellir leur surface.

Le terme dragéification dure employé dans la présente invention comprend également les techniques très voisines que sont le lissage et le givrage. Le lissage consiste en une ou deux applications ou charges d'un sirop cristallisable dilué par rapport à celui utilisé en dragéification dure. Le but est souvent de parfaire l'aspect de surface de produits dragéifiés. Le givrage quant à lui vise également à améliorer l'aspect des produits, mais aussi à isoler ces derniers de l'humidité de l'atmosphère. Cette technique ressemble à une dragéification dure, en ce sens qu'un sirop cristallisable est utilisé. La différence essentielle réside dans le fait que le nombre de cycles réalisés n'est que d'un, deux ou trois seulement.

La dragéification est un procédé long et laborieux, incluant un grand nombre d'étapes successives. Chacune de ces étapes, appelée également cycle de dragéification, inclut typiquement une phase d'application, généralement par pulvérisation, d'un sirop de dragéification (contenant un ou plusieurs polyols, mais aussi parfois des liants comme la gomme arabique ou la gélatine, des colorants comme le $TiO_2$, des édulcorants intenses...) sur les noyaux, une phase rotative de répartition dudit sirop sur les noyaux appelée également temps de pause, et une phase de séchage de chaque nouvelle couche de sirop réalisée par soufflage d'air chaud et sec. Cette succession de cycles doit être répétée un très grand nombre de fois, de l'ordre de dix à quatre-vingt fois, de manière à obtenir le taux de grossissage voulu. L'épaisseur de l'enveloppe ou taux de grossissage est choisie en fonction notamment du noyau à dragéifier ou des effets recherchés. Aujourd'hui, la préoccupation majeure des fabricants des chewing-gums est d'obtenir des chewing-gums possédant une couche dure bien croustillante mais en réduisant les temps de dragéification.

Pendant la première phase de production des centres de chewing-gum, qui consiste à pétrir tous les ingrédients entrant dans la composition à une température comprise entre 50°C et 80°C, la phase liquide et la gomme de base enrobent les édulcorants cristallisés, les dissolvent jusqu'à saturation de la phase liquide. Cependant, comme la température diminue lors du procédé de refroidissement, la solubilité des polyols diminue également et la phase cristalline dissoute va partiellement recristalliser ce qui va conduire à un durcissement du chewing-gum. Ainsi, le rôle de la phase liquide est de contrôler la recristallisation des édulcorants cristallisés afin de prévenir les phénomènes de fragilité ou de durcissement excessif des chewing-gums pendant la production mais aussi pendant le stockage. Si le sirop d'anti-cristallisation contient une quantité significative de polyols dissous similaires à ceux de la phase cristalline, la cristallisation pendant le procédé de production ou durant le stockage aura lieu et conduire à des chewing-gums trop fragiles ou trop durs.

L'eau du chewing-gum peut être apportée sous forme d'eau libre ou par d'autres constituants. La composition de chewing-gum selon l'invention peut comprendre un agent de liaison, dans une concentration de 0,1 % à 30 %. Celui-ci peut être choisi de préférence parmi l'eau, la glycérine, les sirops de mono, di, oligo ou polysaccharides, hydrogénés ou non, et les sirops d'agents de charge hypocaloriques et les mélanges quelconques d'entre eux.

Les sirops de mono, di, oligo ou polysaccharides peuvent être par exemple des sirops de xylitol, de sorbitol, de maltitol, de lactitol, d'isomaltulose, d'isomaltulose hydrogéné, d'érythrose, d'érythritol, des sirops, de préférence hydrogénés, issus de l'hydrolyse d'amidons ou d'inulines, contenant des oligosaccharides et/ou des polysaccharides. Quant aux sirops d'agents de charge hypocaloriques, on préfère retenir en particulier les sirops de polydextrose, de polyglucose, de dextrine.

Selon un mode de réalisation préférentiel, la composition de chewing-gum peut contenir jusqu'à 20% d'un sirop de maltitol.

A titre d'exemple, on peut citer les sirops de maltitol commercialisés par la Demanderesse sous la marque LYCASIN®, comme le LYCASIN® 80/55 (75% de matière sèche et 50-55% en matière sèche de maltitol), le LYCASIN® 85/55 (85% de matière sèche et 50-55% en matière sèche de maltitol). Ces sirops ou agents anti-cristallisation prêts à l'emploi sont particulièrement adaptés pour une utilisation conjointe avec tous les polyols cristallisés cités ci-dessous, et permettent ainsi d'apporter une plasticité améliorée au chewing-gum. L'invention sera mieux comprise à la lecture des exemples suivants, qui ne sauraient limiter en rien la présente invention.

**Exemple 1 : Mesure des surfaces spécifiques de différents agents de charge selon l'invention**

Selon l'invention, la surface spécifique est mesurée soit sur la poudre totale constituant ledit agent de charge, soit sur un échantillon de poudre obtenu sur une coupe de 250 $\mu$m à 841 $\mu$m.

Préparation de l'échantillon

Pour préparer l'échantillon, il faut tamiser une quantité suffisante d'échantillon sur les tamis de 841 $\mu$m et 250 $\mu$m afin de récupérer environ 3 grammes d'une coupe granulométrique comprise entre 841 et 250 microns.

Dans une cellule de mesure de l'appareil, préalablement séchée et tarée à 0,001 g près, introduire une prise d'essai suffisante pour remplir le réservoir de la cellule au ¾.

Dégazage

Placer la cellule contenant l'échantillon au poste de dégazage.
Effectuer celui-ci en se reportant au manuel d'utilisation de l'appareil.

Analyse de la poudre

Le dégazage effectué, repeser la cellule à 0,001 g près et la placer au poste de mesure. Effectuer l'analyse en se reportant au manuel d'utilisation de l'appareil.
L'appareil traite automatiquement les résultats recueillis. Le résultat est exprimé en $m^2/g$.

Tableau 1 : Mesures sur produit avant subi au préalable une coupe sur 250 um à 841 um

| Agent de charge | Surface spécifique(en $m^2/g$) |
|---|---|
| Xylitol 90 Granulométrie 100 $\mu$m | 0.20 |
| Isomalt PF | 0.39 |
| Maltodextrines branchées Nutriose FB06 | 0.21 |
| Allulose | < 0,20 |
| Mannitol 60 | 0.25 |
| Dextrose anhydre | < 0,20 |
| Glucidex 19 | 0.25 |
| Sucre glace amylacée 2% | 0.35 |
| Sorbitol convenant pour l'invention | 0.1 à 0.3 |

**Exemple 2 :**

Caractéristiques d'un agent de charge de type sorbitol selon l'invention

Le tableau 2 ci-dessous reprend les caractéristiques d'un agent de charge de type sorbitol susceptible d'être utilisé dans la fabrication des compositions de chewing-gums selon l'invention.
Ce dernier est comparé à un sorbitol poudre du commerce commercialisé par la Société Demanderesse sous la marque Neosorb®.

Tableau 2 : Caractéristiques d'un agent de charge de type sorbitol LabA

| | Agent de charge de type sorbitol : LabA | Sorbitol Neosorb® P60W |
|---|---|---|
| Sorbitol (%/sec) | 95,5 | 98,5 |
| Maltitol (%/sec) | 1,4 | 0,2 |
| Mannitol (%/sec) | 0,8 | 0,6 |
| Diamètre moyen volumique ($\mu$m) | 320 | 290 |
| Surface spécifique ($m^2/g$) | 0,3 | 0,85 |
| Porosité ml/g | 0.006 | 0,00136 |

**Exemple 3**

Composition de chewing-gum selon l'invention réalisée à partir de l'agent de charge de type sorbitol décrit dans l'exemple 2 ci-dessus.
Le témoin a été réalisé avec un sorbitol poudre commercialisé par la Société Demanderesse sous la marque Neosorb®, de type Neosorb® P60W.

Tous les pourcentages exprimés le sont par rapport au poids sec total de la composition de chewing-gum mise en oeuvre.

## 1. Préparation des compositions de chewing-gums

Ingrédients mis en oeuvre dans les compositions de chewing-gum, tableau 3 ci-dessous:

| Ingrédients | Composition de chewing-gum témoin (%) | Composition de chewing-gum selon l'invention (%) |
|---|---|---|
| Gomme de base Solsona T | 30 | 22 |
| Sorbitol NEOSORB® P60W | 48,270 | 0 |
| Agent de charge de type sorbitol LabA | 0 | 48,693 |
| Sirop de maltitol Lycasin® 85/55 | 4,83 | 14,00 |
| Mannitol 60, poudre fine | 8 | 8 |
| Glycérine | 4 | 3 |
| Arôme liquide menthe fraîche M0060167 | 1,4 | 1,073 |
| Menthol crystal Mane | 0,5 | 0,383 |
| Arôme poudre menthe fraîche SDM0060167 | 2,2 | 2,2 |
| Acesulfame K | 0,150 | 0,150 |
| Aspartame | 0,150 | 0,150 |
| Emulsifiant de type lécithine de tournesol liquide | 0.300 | 0,150 |
| Poudre TIO2 | 0,2 | 0,2 |
| **TOTAL** | **100** | **100** |

La gomme de base SOLSONA T est commercialisée par la société CAFOSA
Le Sorbitol NEOSORB® P60W est une poudre de sorbitol cristalline, commercialisée par la Demanderesse. Le Mannitol 60, Xylitol 90 et le sirop de maltitol Lycasin® 85/55 sont également commercialisés par la Demanderesse.
Tous les arômes sont fournis par la société Mane et fils.

Mode opératoire pour la préparation des compositions de chewing-gum témoin et selon l'invention

- Mélange : procédure en minutes - Réalisé dans un pétrin bras en Z à 45°C - Fabrication de batch de 50 kg de centre

0 min : Introduire la gomme de base fondue (étuvée une nuit à 50°C) et le mannitol.

3 min : Ajouter le Lycasin® 85/55.

5 min : Ajouter l'agent de charge, donc soit le sorbitol NEOSORB®, soit le sorbitol de type LabA.

9 min : Ajouter la glycérine.

10 min : Ajouter l'arôme menthe en poudre et l'arôme de type menthol crystal.

12 min : Ajouter l'arôme de menthe liquide.

15 min : Décharger le pétrin (la pâte est à environ 50°C). Former des pains d'environ 2 kg et les stocker 1 heure à 50% d'HR et à 20°C. Les pains doivent être à environ 48°C pour l'extrusion.

- Extrusion (Appareil Togum TO - E82)

- Consigne de température du corps = 36°C

- Consigne de température de la tête = 39°C.

- Laminage 4 postes - Prédécoupe 2 postes (Appareil Togum TO - W191)

- Saupoudrage de la bande de chewing-gum avec un mélange 90/10 mannitol/talc.

- Maturation

- Stocker les plaques prédécoupées de coussinets à environ 15°C-50% HR pendant environ 24h.

## 2. *Evaluation des qualités organoleptiques des chewing-gums*

Les chewing-gums obtenus précédemment ont été dégustés par un panel de 15 personnes entraînées à la dégustation et à la notation des chewing-gums.

Le panel a été invité à noter de 0 à 4 la souplesse des chewing-gums lors des premières secondes de mâche mais également après trois minutes de mastication.

4 étant le maximum de souplesse et 0 correspondant à un chewing-gum très dur voire cassant.

Le panel a également été invité à noter la perception de l'arôme pendant la mastication. Ce test a également été fait lors des premières secondes de mâche et après trois minutes de mastication afin d'évaluer la persistance de l'arôme.

4 étant la note donnée pour un arôme très fort et 0 correspondant à un chewing-gum ne possédant plus du tout d'arôme.

Les produits étaient présentés dans un ordre aléatoire, et codés avec un numéro à 3 chiffres afin que les panélistes ne soient pas influencés ni par la connaissance des produits ni par leurs codes. Les dégustations ont été faites dans un laboratoire d'analyse sensorielle.

A T+0, le chewing-gum est introduit dans la cavité buccale et en même temps le chronomètre est déclenché. Puis la mastication débute.

Le traitement des données a été effectué par un traitement statistique (ANOVA et tests de comparaison de moyennes sont effectués sur les moyennes obtenues à chaque intervalle de temps).

Tableau 4 : Comparaison de la souplesse et de la persistance de l'arôme lors de la mastication

|  | Evaluation de la souplesse | | Evaluation de la persistance de l'arôme | |
|---|---|---|---|---|
|  | T=10 secondes | T=3 min | T=10 secondes | T=3 min |
| **CG témoin** | 4 | 3 | 4 | 2 |
| **CG selon l'invention** | 4 | 3 | 4 | 3 |

Il ressort que :

- la souplesse des deux chewing-gums évolue de manière identique. Bien que possédant une quantité inférieure de gomme de base que le chewing-gum témoin, aucune différence en terme de texture et plus particulièrement en terme de souplesse entre les deux échantillons n'a été perçue par le panel de dégustateurs.

- Au niveau de la perception et de la persistance de l'arôme, là encore il n'y a pas de différence à t=10 secondes entre le chewing-gum témoin et celui selon l'invention. Bien que possédant une quantité moins importante d'arômes dans la recette, la perception de ce dernier dans le chewing-gum renfermant la composition édulcorante selon l'invention est identique au chewing-gum témoin. Il semblerait même que la persistance de l'arôme soit améliorée dans le temps puisque le chewing-gum obtenu à partir de la composition édulcorante selon l'invention, contenant moins de gomme de base et moins d'arôme est pourtant noté comme étant légèrement meilleur que le chewing-gum témoin au bout de trois minutes de mastication.

Ainsi le chewing-gum fabriqué avec la composition édulcorante selon l'invention et contenant 8% de gomme de base de moins sur poids, soit une réduction de cette dernière de 27%, et contenant 0,44% d'arôme de moins sur poids, soit une réduction de 23% de la quantité d'arôme est identique en termes de texture et est légèrement supérieur en termes de persistance d'arômes.

L'intérêt d'une composition de chewing-gum selon la présente invention est parfaitement démontré par cet exemple.

## Exemple 4 (ne faisant pas partie de l'invention)

Compositions de chewing-gums ne faisant pas partie de l'invention, réalisée à partir de différents agents de charge susceptibles de rentrer dans les dites compositions.

Tous les pourcentages exprimés le sont par rapport au poids sec total de la composition de chewing-gum mise en oeuvre.

Les différentes recettes de composition sont présentées dans le tableau 6 ci-après.

Le témoin est une composition de chewing-gum réalisée avec un sorbitol poudre du commerce commercialisé par la Société Demanderesse sous la marque Neosorb®, mais possédant une surface spécifique supérieure à 0,5 m$^2$/g et étant le sorbitol généralement utilisé dans l'art antérieur.

| Agent de charge | Composition témoin | Sucre glace | Dextrose | Fructose | Allulose |
|---|---|---|---|---|---|
| Allulose poudre broyée 125 microns | - | - | - | - | 61,90 |
| Sucre glace | - | 62,05 | - | - | - |
| Dextrose | - | - | 62,05 | - | - |
| Fructose | | - | - | 62,05 | - |
| Sorbitol NEOSORB® P60W | 59,6 | | | | |
| Gum base Solsona T | 30 | 22,00 | 22,00 | 22,00 | 22,00 |
| Glycérine | 1 | 1,00 | 1,00 | 1,00 | 1,00 |
| Sirop de glucose à 83%MS | 7,5 | 13,50 | 13,50 | 13,50 | 13,50 |
| Arôme menthe liquide | 1,3 | 0,95 | 0,95 | 0,95 | 0,95 |
| Arôme menthe en poudre | 0,6 | 0,50 | 0,50 | 0,50 | 0,50 |
| Aspartame | - | - | | | 0,15 |
| **TOTAL** | **100** | **100** | **100** | **100** | **100** |
| **% de réduction de gomme de base** <br> **- sur poids** <br> **- sur gomme de base** | **-** <br><br> | <br> **8** <br> **27** | <br> **8** <br> **27** | <br> **8** <br> **27** | <br> **8** <br> **27** |
| **% de réduction d'arômes** <br> **- sur poids** <br> **- sur gomme de base** | **-** <br><br> | <br> **0,45** <br> **24** | <br> **0,45** <br> **24** | <br> **0,45** <br> **24** | <br> **0,45** <br> **24** |

Le procédé de préparation des centres de chewing-gum est tel que celui décrit dans l'exemple précédent.

Les chewing-gums obtenus précédemment ont été dégustés par un panel de 15 personnes entraînées à la dégustation et à la notation des chewing-gums.

La souplesse et la perception aromatique ont été notées selon le même protocole que celui décrit dans l'exemple précédent.

Tous les chewing-gums dégustés présentaient une souplesse satisfaisante et une perception aromatique identique.

Ainsi, il est tout à fait possible de réaliser des compositions de chewing-gums possédant une quantité de gomme de base et d'arômes réduites sans impacter sur les qualités organoleptiques finales des produits obtenus dès lors qu'on utilise un agent de charge possédant une surface spécifique faible.

Les compositions présentent toutes les 4 une réduction de gomme de base de 8% sur poids, soit une réduction de cette dernière de 27%, et une réduction de 0,45% d'arôme sur poids, soit une réduction de 24% de la quantité d'arôme.

Cela permet des gains économiques considérables sans impact négatif sur le produit. Ce qui actuellement recherché par le producteur de chewing-gums.

**Revendications**

1. Composition de chewing-gum **caractérisée en ce qu'**elle comporte entre 30% et 70% en poids d'un agent de charge différent du maltitol possédant une surface spécifique déterminée par la méthode BET inférieure à 0,5 m$^2$/g, de préférence comprise entre 0,1 et 0,45 m$^2$/g, ladite surface spécifique étant mesurée sur une coupe de 250$\mu$m à 841$\mu$m, les pourcentages étant exprimés en poids par rapport au poids total de la composition de chewing-gum mise en oeuvre, le dit agent de charge étant le sorbitol.

2. Composition de chewing-gum selon la revendication précédente, **caractérisée en ce que** l'agent de charge possède une surface spécifique déterminée par la méthode BET comprise entre 0,2 et 0,45 m$^2$/g, et de préférence entre 0,2 et 0,3 m$^2$/g, ladite surface spécifique étant mesurée sur une coupe de 250$\mu$m à 841$\mu$m.

3. Composition de chewing-gum selon la revendication 1, **caractérisée en ce que** qu'elle comprend un agent de charge possédant une porosité inférieure à 0,0085 ml/g, de préférence inférieure à 0,0080 ml/g et encore plus préférentiellement inférieure à 0,0070 ml/g.

4. Composition selon la revendication 1, **caractérisée en ce qu'**elle comporte entre 40% et 60%, et plus préférentiellement encore entre 45% et 55% d'agent de charge, les pourcentages étant exprimés en poids par rapport au poids total de la composition de chewing-gum mise en oeuvre.

5. Composition selon la revendication précédente, **caractérisée en ce que** le sorbitol présente une pureté supérieure à 95% en poids sec de sorbitol.

6. Composition de chewing-gum selon la revendication 1 **caractérisée en ce qu'**elle comprend :

   - de 10% à 28%, préférentiellement de 15% à 25%, et encore plus préférentiellement 20% d'au moins une gomme de base,
   - de 30% à 70%, de préférence de 40% à 60%, et plus préférentiellement encore de 45% à 55% d'un agent de charge différent du maltitol possédant une surface spécifique faible, c'est-à-dire inférieure à 0,5 m$^2$/g, mesurée sur une coupe de 250$\mu$m à 841$\mu$m
   - de 0,1 % à 5 %, préférentiellement de 0,5 % à 3 %, et encore plus préférentiellement de 1 à 1,8 % d'au moins un arôme,

   les pourcentages étant indiqués en poids sec par rapport au poids sec total de la dite composition, le dit agent de charge étant le sorbitol.

**Patentansprüche**

1. Kaugummi-Zusammensetzung, **dadurch gekennzeichnet, dass** sie zwischen 30 Gewichts-% und 70 Gewichts-% eines von Maltit unterschiedlichen Füllstoffs umfasst, der eine spezifische Oberfläche, bestimmt nach der BET-Methode, von kleiner als 0,5 m$^2$/g, bevorzugt zwischen 0,2 und 0,45 m$^2$/g aufweist, wobei die spezifische Oberfläche über einen Schnitt von 250 $\mu$m bis 841 $\mu$m gemessen wird, wobei die Prozentangaben als Gewicht bezogen auf das Gesamtgewicht der hergestellten Kaugummi-Zusammensetzung angegeben sind, wobei der Füllstoff Sorbit ist.

2. Kaugummi-Zusammensetzung gemäß dem vorhergehenden Anspruch, **dadurch gekennzeichnet, dass** der Füllstoff eine spezifische Oberfläche, bestimmt nach der BET-Methode, zwischen 0,2 und 0,45 m$^2$/g, und bevorzugt zwischen 0,1 und 0,3 m$^2$/g aufweist, wobei die spezifische Oberfläche über einen Schnitt von 250 $\mu$m bis 841 $\mu$m gemessen wird.

3. Kaugummi-Zusammensetzung gemäß Anspruch 1, **dadurch gekennzeichnet, dass** sie einen Füllstoff umfasst, der eine Porosität von kleiner als 0,0085 ml/g, bevorzugt von kleiner als 0,0080 ml/g und noch stärker bevorzugt von keiner als 0,0070 ml/g aufweist.

4. Zusammensetzung gemäß Anspruch 1, **dadurch gekennzeichnet, dass** sie zwischen 40% und 60% und noch stärker bevorzugt zwischen 45% und 55% an Füllstoff umfasst, wobei die Prozentangaben als Gewicht bezogen auf das Gesamtgewicht der hergestellten Kaugummi-Zusammensetzung angegeben sind.

5. Zusammensetzung gemäß dem vorhergehenden Anspruch, **dadurch gekennzeichnet, dass** der Sorbit eine Reinheit von größer als 95% bezogen auf das Trockengewicht an Sorbit aufweist.

6. Kaugummi-Zusammensetzung gemäß Anspruch 1, **dadurch gekennzeichnet, dass** sie umfasst:

- 10% bis 28%, bevorzugt 15% bis 25% und noch stärker bevorzugt 20% wenigstens einer Gummi-Basis,
- 30% bis 70%, bevorzugt 40% bis 60% und noch stärker bevorzugt 45% bis 55% eines von Maltit unterschiedlichen Füllstoffs, der eine kleine spezifische Oberfläche aufweist, das heißt kleiner als 0,5 m$^2$/g, gemessen über einen Schnitt von 250 $\mu$m bis 841 $\mu$m,
- 0,1 bis 5%, bevorzugt 0,5 bis 3% und noch stärker bevorzugt 1 bis 1,8% wenigstens eines Aromas,

wobei die Prozentangaben als Trockengewicht bezogen auf das Gesamttrockengewicht der Zusammensetzung angegeben sind, wobei der Füllstoff Sorbit ist

## Claims

1. A chewing gum composition **characterized in that** it comprises between 30% and 70% by weight of a bulking agent other than maltitol having a specific surface area, determined by the BET method, of less than 0.5 m$^2$/g, preferably of between 0.1 and 0.45 m$^2$/g, said specific surface area being measured on a fraction of 250 $\mu$m to 841 $\mu$m, the percentages being expressed by weight relative to the total weight of the chewing gum composition used, said bulking agent being sorbitol.

2. The chewing gum composition as claimed in the preceding claim, **characterized in that** the bulking agent has a specific surface area, determined by the BET method of between 0.2 and 0.45 m$^2$/g, and preferably between 0.2 and 0.3 m$^2$/g, said specific surface area being measured on a fraction of 250 $\mu$m to 841 $\mu$m.

3. The chewing gum composition as claimed in claim 1, **characterized in that** it comprises a bulking agent having a porosity of less than 0.0085 ml/g, preferably less than 0.0080 ml/g and even more preferentially less than 0.0070 ml/g.

4. The composition as claimed in claim 1, **characterized in that** it comprises between 40% and 60%, and even more preferentially between 45% and 55% of bulking agent, the percentages being expressed by weight relative to the total weight of the chewing gum composition used.

5. The composition as claimed in the preceding claim, **characterized in that** the bulking agent is sorbitol, preferably having a purity of greater than 95% by dry weight of sorbitol.

6. The chewing gum composition as claimed in claim 1, **characterized in that** it comprises:

- from 10% to 28%, preferentially from 15% to 25%, and even more preferentially 20%, of at least one gum base,
- from 30% to 70%, preferably from 40% to 60%, and even more preferentially from 45% to 55%, of a bulking agent other than maltitol having a low specific surface area, i.e. less than 0.5 m$^2$/g, measured on a fraction of 250 $\mu$m to 841 pm,
- from 0.1% to 5%, preferentially from 0.5% to 3%, and even more preferentially from 1% to 1.8%, of at least one flavoring,

the percentages being indicated by dry weight relative to the total dry weight of said composition, said bulking agent being sorbitol.

**EP 3 164 013 B1**

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Documents brevets cités dans la description

- EP 0664960 B
- EP 0347121 A
- FR 1456288
- WO 2005060944 A

### Littérature non-brevet citée dans la description

- **S. BRUNAUER et al.** *Journal of American Chemical Society,* 1938, vol. 60, 309